(19) Europäisches Patentamt
European Patent Office
Office européen des brevets

(11) Numéro de publication: **0 168 309**
**B1**

(12) **FASCICULE DE BREVET EUROPEEN**

(45) Date de publication du fascicule du brevet: **09.03.88**

(21) Numéro de dépôt: **85401234.1**

(22) Date de dépôt: **20.06.85**

(51) Int. Cl.[4]: **C 07 D 498/04,** A 61 K 31/435, C 07 D 215/56 // (C07D498/04, 261:00, 221:00)

(54) **Nouveaux dérivés de l'isoxazoloquinoléinone, procédé et intermédiaires de préparation, application à titre de médicaments, et compositions les renfermant.**

(30) Priorité: **25.06.84 FR 8409961**

(43) Date de publication de la demande: **15.01.86 Bulletin 86/3**

(45) Mention de la délivrance du brevet: **09.03.88 Bulletin 88/10**

(84) Etats contractants désignés: **AT BE CH DE FR GB IT LI LU NL SE**

(56) Documents cités: **FR - A - 2 537 140**

(73) Titulaire: **ROUSSEL-UCLAF, 35, boulevard des Invalides, F-75007 Paris (FR)**

(72) Inventeur: **Humbert, Daniel, 15, Gaston Charle, F-94120 Fontenay-sous-Bois (FR)**
Inventeur: **Gasc, Jean-Claude, 6, place Georges Lyssandre, F-93140 Bondy (FR)**
Inventeur: **Hunt, Peter Francis, 18, rue de l'Hôtel-Dieu, F-95500 Gonesse (FR)**

(74) Mandataire: **Tonnellier, Marie-José et al, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 168 309 B1

**Description**

La présente invention concerne de nouveaux dérivés de l'isoxazoloquinoléinone, le procédé de préparation, l'application à titre de médicaments de ces nouveaux dérivés, les compositions les renfermant et de nouveaux intermédiaires.

L'invention a pour objet de nouveaux dérivés caractérisés en ce qu'ils répondent à la formule générale I

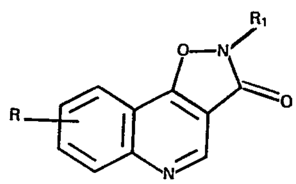

(I)

dans laquelle R représente un atome d'hydrogène, de fluor, de chlore ou de brome, un radical alkyle linéaire renfermant de 1 à 5 atomes de carbone, ou ramifié renfermant de 3 à 5 atomes de carbone, un radical alkoxy renfermant de 1 à 5 atomes de carbone, un radical nitro ou trifluorométhyle et $R_1$ représente un radical phényle éventuellement substitué par un radical alkyle linéaire renfermant de 1 à 5 atomes de carbone, ou ramifié renfermant de 3 à 5 atomes de carbone, par un radical alkoxy renfermant de 1 à 5 atomes de carbone, ou par un atome d'halogène.

Dans la formule générale I et dans ce qui suit, le terme radical alkyle linéaire renfermant de 1 à 5 atomes de carbone désigne, de préférence, un radical méthyle ou éthyle; le terme radical alkyle ramifié renfermant de 3 à 5 atomes de carbone désigne, de préférence, un radical isopropyle ou isobutyle; le terme radical alkoxy renfermant de 1 à 5 atomes de carbone désigne, de préférence, un radical méthoxy, éthoxy ou propoxy. Lorsque $R_1$ représente un radical phényle substitué, le radical phényle peut être plurisubstitué ou, de préférence, monosubstitué et les substituants peuvent être en position 3-4, 2 ou 3 par exemple, et de préférence, en position 4; le terme atome d'halogène désigne, par exemple, un atome d'iode ou de fluor, mais de préférence, de chlore ou de brome.

Parmi les produits objet de l'invention, on peut citer notamment les dérivés répondant à la formule générale I ci-dessus, caractérisés en ce que dans ladite formule R représente un atome d'halogène ou de chlore.

Parmi ceux-ci, on peut citer tout particulièrement la 2-phényl isoxazolo (4,5-c) quinoléin-3(2H)-one.

On peut citer également:

la 8-fluoro 2-phényl isoxazolo (4,5-c) quinoléin-3-(2H)-one, et

la 8 méthoxy 2-phényl isoxazolo (4,5-c) quinoléin-3(2H)-one.

L'invention a également pour objet un procédé de préparation des dérivés, tels que définis par la formule générale I ci-dessus, caractérisé en ce que l'on fait réagir un dérivé de formule II

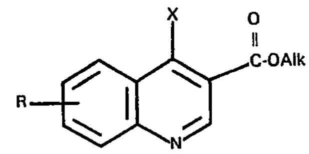

(II)

dans laquelle Alk représente un radical méthyle, éthyle ou propyle, X représente un atome de chlore ou de brome et R a la signification déjà indiquée, avec un dérivé de l'hydroxylamine de formule III

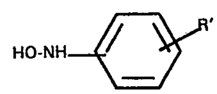

(III)

dans laquelle R' a la signification déjà indiquée pour les substitutions optionnelles du radical phényle, pour obtenir un dérivé de formule générale I que l'on isole.

Dans des conditions préférentielles de mise en oeuvre de l'invention, le procédé de préparation ci-dessus décrit est caractérisé en ce que:

Alk représente un radical éthyle

X représente un atome de chlore.

La réaction du dérivé de formule II avec l'hydroxylamine de formule III a lieu de préférence dans un milieu alcalin, tel que l'hydrure de sodium dans le tétrahydrofuranne.

Les produits de formule II sont des produits connus. Les dérivés de formule III sont également connus ou peuvent être préparés comme indiqué dans Organique Synthèse Vol. I, p. 445 ou dans J.A.C.S. (1956), 78, p. 336.

L'invention a également pour objet un procédé de préparation des dérivés tels que définis par la formule générale I ci-dessus, caractérisé en ce que l'on fait réagir un dérivé de formule IV

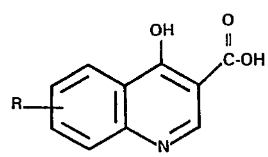

(IV)

dans laquelle R a la signification déjà indiquée, avec un dérivé de l'hydroxylamine de formule III, telle que définie précédemment, pour obtenir un dérivé de formule V

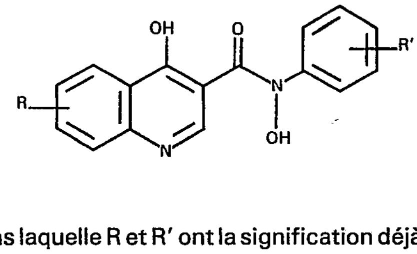

(V)

dans laquelle R et R' ont la signification déjà indiquée, que l'on cyclise pour obtenir un dérivé de formule générale I.

Dans des conditions préférentielles de mise en oeuvre de l'invention, le procédé de préparation ci-dessus décrit est caractérisé en ce que:

l'acide de formule IV est utilisé sous forme de chlorure d'acide, préparé, par exemple, par action du chlorure de thionyle;

la cyclisation du dérivé de formule V est effectuée en présence de triphénylphospine et d'azodicarboxylate d'éthyle, dans un solvant, tel que le diméthylformamide, le chlorure de méthylène, le toluène.

Les dérivés de formule IV sont connus. Ils peuvent être préparés par exemple à partir des esters d'alcoyle correspondants, décrits dans la demande européenne 67 772.

Les dérivés objet de la présente invention, possèdent de très intéressantes propriétés pharmacologiques; ils sont doués notamment d'une remarquable affinité pour les récepteurs de benzodiazépines.

Ces propriétés sont illustrées plus loin dans la partie expérimentale.

Ces propriétés justifient l'utilisation des dérivés de l'isoxazolo quinoléinone à titre de médicaments.

La présente demande a ainsi également pour objet l'application, à titre de médicaments, des dérivés de l'isoxazolo quinoléinone tels que définis par la formule générale I.

Parmi ceux-ci, on retient notamment ceux répondant à la formule générale I, dans laquelle R représente un atome d'halogène ou de chlore.

Parmi ces derniers, on retient tout particulièrement la 2-phényl isoxazolo (4,5-c) quinoléin-3(2H)-one, ainsi que la 8-fluoro 2-phényl isoxazolo (4,5-c) quinoléin-3(2H)-one et la 8-méthoxy 2-phényl isoxazolo (4,5-c) quinoléin-3(2H)-one.

Les médicaments selon l'invention trouvent leur emploi, par exemple, dans le traitement des états anxieux tels que l'anxiété chronique, associée ou non à de l'insomnie ou à des troubles du comportement, de l'angoisse chez l'adulte ou l'enfant, ou en complément, lors des traitements par neuroleptiques ou antidépresseurs dans les états psychotiques ou dépressifs.

La dose usuelle, variable selon le dérivé utilisé, le sujet et l'affection en cause peut être par exemple de 1 mg à 500 mg par jour. Par voie orale, chez l'homme, le dérivé de l'exemple 1 peut être administré à la dose quotidienne de 2 mg à 200 mg, par exemple pour le traitement de l'anxiété chronique, soit environ de 0,03 mg à 3 mg par kilogramme de poids corporel.

L'invention a également pour objet les compositions pharmaceutiques qui renferment au moins un dérivé précité, à titre de principe actif.

A titre de médicaments, les dérivés répondant à la formule générale I peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a enfin pour objet, les dérivés de formule V, tels que définis précédemment.

Les exemples qui suivent illustrent la présente invention, sans toutefois la limiter.

*Exemple 1*

*2-phényl isoxazolo (4,5-c) quinoléin-3(2H)-one*

On ajoute goutte à goutte à 0°C, sous agitation et atmosphère inerte une solution de 9 g de N-phénylhydroxylamine décrite dans Organique Synthèse Vol. I, p. 445 à une suspension de 3,85 g d'hydrure de sodium à 50% dans 100 $cm^3$ de tétrahydrofuranne, laisse sous agitation à 5°C pendant 300 mn, ajoute à 0°C 18,8 g de 4-chloro 3-quinoléine carboxylate d'éthyle dans 100 $cm^3$ de tétrahydrofuranne et laisse sous agitation pendant 16 heures à température ambiante. On concentre sous pression réduite, reprend le résidu dans de la glace, extrait au chlorure de méthylène, lave, sèche, concentre, purifie par chromatographie sur silice (Eluat: acétate d'éthyle), et récupère 1,2 g du produit attendu (correspondant à la 3ème fraction éluée) F ≃ 163°C après recristallisation dans l'éther isopropylique.

Analyse pour $C_{16}H_{10}N_2O_2$ = 262,269

| | | | |
|---|---|---|---|
| Calculé: | C 73,27 | H 3,84 | N 10,68% |
| Trouvé: | C 72,9 | H 3,8 | N 10,5 % |

*Exemple 2*

*8-fluoro 2-phényl isoxazolo (4,5-c) quinoléin-3-(2H)-one*

*Stade A:* N,4-dihydroxy 6-fluoro N-phényl 3-quinoléine carboxamide

On porte au reflux et sous agitation pendant 16 heures 6,2 g d'acide 4-hydroxy 6-fluoro 3-quinoléine carboxylique, 100 $cm^3$ de benzène anhydre et 14,28 g de chlorure de thionyle. On refroidit, essore le chlorure d'acide, le lave au benzène et le sèche sous pression réduite à température ambiante. En agitant, on ajoute ce produit par petites fractions à un mélange refroidi à 0°C, contenant 10 g de N-phényl--hydroxylamine, 4,8 $cm^3$ de pyridine et 50 $cm^3$ de dichlorométhane. On agite à température ambiante pendant 16 heures. On essore le précipité, le lave à l'eau jusqu'à élimination des ions $Cl^{\ominus}$. On obtient 6,68 g de produit attendu après recristallisation du produit brut dans le diméthylformamide. F = 260°C.

*Stade B:* 8-fluoro 2-phényl isoxazolo (4,5-c) quinoléin-3(2H)-one

Dans un mélange de 8,81 g de triphénylphosphine et 50 $cm^3$ de diméthylformamide, on verse à 10-15°C, 5,85 g d'azodicarboxylate d'éthyle et poursuit l'agitation pendant 15 minutes puis ajoute par petites fractions et à 0°C, 6,68 g du produit obtenu ci-dessus. On maintient l'agitation pendant 2 heures à 0°C et pendant 16 heures à température ambiante. On essore le produit cristallisé, vers le filtrat dans l'eau glacée, extrait au dichlorométhane, lave la phase organique à l'eau, sèche et élimine le solvant. On chromatographie le résidu sur silice en éluant par un mélange chlorure de méthylène-acétate d'éthyle (9-1). On obtient 0,92 g de produit attendu. F = 174°C.

*Exemple 3*

*7-méthoxy 2-phényl isoxazolo (4,5-c) quinoléin--3(2H)-one*

*Stade A:* N,4-dihydroxy 7-méthoxy N-phényl -3-quinoléine carboxamide

On opère comme au stade A de l'exemple 2 à partir de 5,48 g d'acide 4-hydroxy 7-méthoxy 3-quinoléine carboxylique et obtient 4,05 g de produit attendu. F = 260°C.

*Stade B:* 7-méthoxy 2-phényl isoxazolo (4,5-c) quinoléin-3-(2H)-one

On opère comme au stade B de l'exemple 2 à partir de 3,86 g de N,4-dihydroxy 7-méthoxy N-phényl 3--quinoléine carboxamide. Après 18 heures à température ambiante, on verse le mélange réactionnel dans l'eau et extrait les produits qui cristallisent par du dichlorométhane. On lave la phase organique à l'eau et élimine le solvant. On chromatographie le résidu sur silice en éluant par un mélange chlorure de méthylène-acétate d'éthyle (9-1) et obtient 0,7 g de produit attendu. F = 170°C.

*Exemple 4*

*8-méthoxy 2-phényl isoxazolo (4,5-c) quinoléin--3(2H)-one*

*Stade A:* N,4-dihydroxy 6-méthoxy N-phényl 3-quinoléine carboxamide

On opère comme au stade A de l'exemple 2 à partir de 5,48 g d'acide 4-hydroxy 6-méthoxy 3-quinoléine carboxylique et obtient 3,06 g de produit attendu après recristallisation dans l'acide acétique. F = 226°C.

*Stade B:* 8-méthoxy 2-phényl isoxazolo (4,5-c) quinoléin-3(2H)-one

On opère comme au stade B de l'exemple 2 à partir de 2,92 g du produit obtenu ci-dessus. Après 18 heures d'agitation, on essore l'insoluble, vers le filtrat dans l'eau glacée, extrait les cristaux formés par du chlorure de méthylène, lave la phase organique à l'eau, la sèche et élimine les solvants. On chromatographie le résidu sur silice en éluant par un mélange chlorure de méthylène-acétate d'éthyle (9-1). On obtient 0,9 g de produit attendu. F = 140°C.

*Exemple 5*

*8-trifluorométhyl 2-phényl isoxazolo (4,5-c) quinoléin-3(2H)-one*

*Stade A:* N,4-dihydroxy N-phényl 6-trifluorométhyl 3-quinoléine carboxamide

On opère comme au stade A de l'exemple 2 en partant de 9 g d'acide 4-hydroxy 6-trifluorométhyl 3-quinoléine carboxylique et obtient 5,45 g de produit attendu. F = 260°C (décomposition).

*Stade B:* 8-trifluorométhyl 2-phényl isoxazolo (4,5-c) quinoléin-3(2H)-one

On opère comme au stade B de l'exemple 2 à partir de 5,43 g du produit obtenu ci-dessus et obtient 0,75 g de produit attendu. F = 175°C.

*Exemple 6*

*7-chloro 2-phényl isoxazolo (4,5-c) quinoléin--3(2H)-one*

*Stade A:* 7-chloro N,4-dihydroxy N-phényl 3-quinoléine carboxamide

On opère comme au stade A de l'exemple 2 à partir de 9,6 g d'acide 7-chloro 4-hydroxy 3-quinoléine carboxylique. On concentre la suspension, reprend le résidu à l'eau, essore le précipité, le lave à l'eau jusqu'à élimination des ions $Cl^{\ominus}$ et obtient 10,87 g de produit attendu après recristallisation dans le diméthylformamide. F = 260°C.

*Stade B:* 7-chloro 2-phényl isoxazolo (4,5-c) quinoléin-3(2H)-one

On opère comme au stade B de l'exemple 2 à partir de 9,95 g du produit ci-dessus. Après agitation pendant 18 heures, on essore, chromatographie le filtrat sur silice, élue par un mélange chlorure de méthylène-acétate d'éthyle (9-1) et obtient 2,65 g de produit attendu que l'on cristallise dans l'isopropanol et recueille 1,7 g de produit. F = 172°C.

*Exemple 7*

*8-chloro 2-phényl isoxazolo (4,5-c) quinoléin--3(2H)-one*

*Stade A:* 6-chloro N,4-dihydroxy N-phényl 3-quinoléine carboxamide

On opère comme au stade A de l'exemple 2 à partir de 9 g d'acide 6-chloro 4-hydroxy 3-quinoléine carboxylique et obtient 6,2 g de produit brut.

*Stade B:* 8-chloro 2-phényl isoxazolo (4,5-c) quinoléin-3(2H)-one

On opère comme au stade B de l'exemple 2 à partir de 5,48 g du produit obtenu ci-dessus. Après 18 heures d'agitation, on concentre le solvant sous pression réduite, dilue avec 100 $cm^3$ de dichlorométhane et chromatographie sur silice en éluant par un mélange chlorure de méthylène-acétate d'éthyle (9-1). On récupère 3,46 g de produit impur que l'on cristallise dans l'isopropanol et obtient 350 mg de produit attendu. F = 174°C.

*Exemple 8*

*2-(4-chlorophényl) isoxazolo (4,5-c) quinoléin--3(2H)-one*

*Stade A:* N,4-dihydroxy N-(4-chlorophényl) 3-quinoléine carboxamide

On opère comme au stade A de l'exemple 6 à partir de 9,45 g d'acide 4-hydroxy 3-quinoléine carboxamide et de N-(4-chlorophényl) hydroxylamine et obtient 14,4 g de produit brut attendu.

*Stade B:* 2-(4-chlorophényl) isoxazolo (4,5-c) quinoléin-3(2H)-one

On opère comme au stade B de l'exemple 2 à partir de 6,1 g du produit obtenu ci-dessus. Après 18 heures d'agitation, on filtre un insoluble et chromatographie le filtrat sur silice, élue par un mélange chlorure de méthylène-acétate d'éthyle (9-1). On recueille 4,8 g de produit attendu. Après recristallisation dans l'isopropanol. F = 156°C.

*Exemple 9*

*8-éthyl 2-phényl isoxazolo (4,5-c) quinoléin-3(2H)-one*

*Stade A:* N,4-dihydroxy 6-éthyl N-phényl 3-quinoléine carboxamide

On opère comme au stade A de l'exemple 6 en partant de 3,2 g d'acide 6-éthyl 4-hydroxy 3-quinoléine carboxylique. Après lavage à l'eau du précipité, on le recristallise dans un mélange diméthylformamide-eau (1-1) et obtient 3,48 g de produit attendu. F = 218°C.

*Stade B:* 8-éthyl 2-phényl isoxazolo (4,5-c) quinoléin-3(2H)-one

On opère comme au stade B de l'exemple 2 à partir de 2,68 g de produit obtenu au stade A en refroidissant à −7°C puis on laisse remonter la température à 20°C et agite 24 heures. On essore et chromatographie le filtrat sur silice en éluant par un mélange chlorure de méthylène-acétate d'éthyle (9-1). On obtient 1,53 g de produit attendu. Après recristallisation dans l'isopropanol. F = 136°C.

*Exemple 10*

*2-phényl isoxazolo (4,5-c) quinoléin-3(2H)-one*

*Stade A:* N,4-dihydroxy N-phényl 3-quinoléine carboxamide

On opère comme au stade A de l'exemple 2 à partir de 9,46 g d'acide 4-hydroxy 3-quinoléine carboxylique et obtient 10,4 g de produit attendu. F ≃ 220°C (décomposition).

*Stade B:* 2-phényl isoxazolo (4,5-c) quinoléin-3(2H)-one

On opère comme au stade B de l'exemple 2 à partir de 5,6 g du produit obtenu ci-dessus. Après extraction au chlorure de méthylène, des produits du milieu réactionnel, on chromatographie le résidu sur silice, élue par un mélange acétate d'éthyle-cyclohexane (3-7). On effectue une deuxième chromatographie sur silice en éluant par un mélange chlorure de méthylène-acétate d'éthyle (9-1) et obtient 1,1 g de produit attendu. F = 163°C.

*Exemple 11*

On a préparé des comprimés répondant à la formule:

| | |
|---|---|
| 2-phényl isoxazolo (4,5-c) quinoléin-3(2H)-one | 10 mg; |
| Excipient q.s. pour un comprimé terminé à | 100 mg. |

(Détail de l'excipient: lactose, amidon, talc, stéarate de magnésium).

*ETUDE PHARMACOLOGIQUE*

1) Affinité pour les récepteurs des benzodiazépines

La technique est inspirée de celle de Möhler et Okada: Science, Vol. 198, p. 849-851 (1977).

On homogénéise au vingtième (poids/volume) dans le sucrose 0,32 M, les cortes prélevés sur des cerveaux de rats mâles pesant 150 g en moyenne. Après centrifugation de l'homogénat à 1000 g pendant 10 minutes à 0°C, le surnageant est centrifugé à 30 000 g pendant 20 minutes à 4°C. Le culot est mis en suspension dans 20 volumes de tampon Tris HCl 50 mM pH 7,4 et centrifugué à 30 000 g pendant 20 minutes à 4°C. Le nouveau culot obtenu est mis en suspension dans 50 ml de tampon Krebs Tris HCl pH 7,4.

On fait ensuite incuber pendant 30 minutes à 0°C, 2 ml de suspension en présence de $^{3}H$ diazepam à la concentration $10^{-9}$M:

i) seule,
ii) avec des concentrations croissantes du produit à tester ou,
iii) pour déterminer la fixation non spécifique, avec du diazepam non radoactif à la concentration $10^{-6}$M.

Les suspensions incubées sont filtrées sur Whatman GF/C et les filtres sont lavés par deux fois 5 ml de tampon Krebs Tris HCl pH 7,4 à 0°C.

La radioactivité des filtres est mesurée par scintillation liquide.

L'activité du produit est exprimée en C. I. 50: concentration inhibant 50% de la liaison spécifique de $^{3}H$ diazepam.

Le résultat obtenu est le suivant:

| Produit de l'exemple | C. I. 50 en nM |
|---|---|
| 1 | 120 |
| 2 | 400 |
| 4 | 2460 |

2) Etude de la toxicité aiguë

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants:

| Produit de l'exemple | $DL_0$ en mg/kg |
|---|---|
| 1 | $\geqslant$ 400 |
| 2 | $\geqslant$ 400 |
| 3 | $\geqslant$ 200 |
| 4 | 200 |
| 5 | $\geqslant$ 200 |
| 7 | $\geqslant$ 100 |

3) Action sur le taux sérique de corticostérone après stress au bruit.

*Technique*

Des lots de 5 rats sont constitués la veille de l'essai et placés dans un pièce au calme. Les composés sont administrés par voie orale le lendamain entre 8 et 9 heures du matin. Une heure après, le stress est appliqué: il consiste à changer les animaux de cage, à les placer dans une pièce où un poste de radio fonctionne bruyamment (90 décibels), puis 1/2 heure plus tard, à leur faire une piqûre intrapéritonéale sans injection. Dix minutes après celle-ci, les rats sont anestésiés à l'halothane et des échantillons de sang sont prélevés par décapitation. Les taux de corticostérone sérique sont alors déterminés.

Les résultats sont exprimés en $DE_{50}$, c'est-à-dire en dose de composé testé qui réduit de 50% l'augmentation du taux de corticostérone sérique chez les animaux traités par rapport aux animaux témoins.

Les résultats sont les suivants:

| Dérivé de l'exemple | $DE_{50}$ en mg/kg |
|---|---|
| 2 | < 50 |
| 4 | < 100 |

Le stress est moins important chez les animaux traités; ces animaux ont donc un degré d'anxiété moindre que celui des témoins.

**Revendications**

1. Les dérivés de l'isoxazolo quinoléinone caractérisés en ce qu'ils répondent à la formule générale I

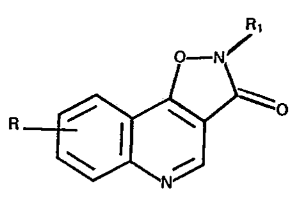

(I)

dans laquelle R représente un atome d'hydrogène, de fluor, de chlore ou de brome, un radical alkyle linéaire renfermant de 1 à 5 atomes de carbone, ou ramifié renfermant de 3 à 5 atomes de carbone, un radical alkoxy renfermant de 1 à 5 atomes de carbone, un radical nitro ou trifluorométhyle et $R_1$ représente un radical phényle éventuellement substitué par un radical alkyle linéaire renfermant de 1 à 5 atomes de carbone, ou ramifié renfermant de 3 à 5 atomes de carbone, par un radical alkoxy renfermant de 1 à 5 atomes de carbone, ou par un atome d'halogène.

2. Les dérivés tels que définis par la formule générale I de la revendication 1, caractérisé en ce que R représente un atome d'hydrogène ou de chlore.

3. La 2-phényl isoxazolo (4,5-c) quinoléin-3(2H)-one.

4. La 8-fluoro 2-phényl isoxazolo (4,5-c) quinoléin-3(2H)-one.

5. La 8-méthoxy 2-phényl isoxazolo (4,5-c) quinoléin-3(2H)-one.

6. Procédé de préparation des dérivés de l'isoxazolo quinoléinone tels que définis par la formule générale I de la revendication 1, caractérisé en ce que l'on fait réagir un dérivé de formule II

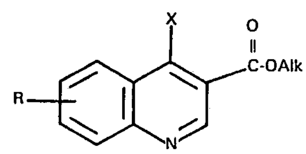

(II)

dans laquelle Alk représente un radical méthyle, éthyle ou propyle, X représente un atome de chlore ou de brome, et R a la significatoin déjà indiquée avec un dérivé de l'hydroxylamine de formule III

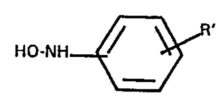

(III)

dans laquelle R' a la signification déjà indiquée à la revendication 1 pour les substitutions optionnelles du radical phényle, pour obtenir un dérivé de formule générale I que l'on isole.

7. Procédé de préparation des dérivés de l'isoxazolo quinoléinone tels que définis par la formule générale I de la revendication 1, caractérisé en ce que l'on fait réagir un dérivé de formule IV

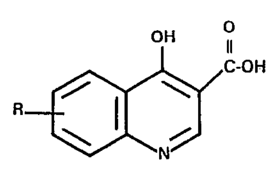

(IV)

dans laquelle R a la signification déjà indiquée, avec un dérivé de l'hydroxylamine de formule III, telle que définie à la revendication 6, pour obtenir un dérivé de formule V

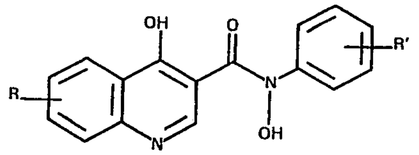

(V)

dans laquelle R et R' ont la signification déjà indiquée, que l'on cyclise pour obtenir un dérivé de formule générale I.

8. Médicaments, caractérisés en ce qu'ils sont constitués par les nouveaux dérivés de l'isoxazolo quinoléinone, tels que définis par la formule générale I de la revendication 1.

9. Médicaments, caractérisés en ce qu'ils sont constitués par les nouveaux dérivés de l'isoxazolo quinoléinone, tels que définis dans l'une des revendications 2 à 5.

10. Compositions pharmaceutiques, caractérisés en ce qu'elles renferment, à titre de principe actif, l'un au moins des médicaments, tels que définis à l'une des revendications 8 ou 9.

11. Les produits de formule V, telle que définie à la revendication 7.

**Patentansprüche**

1. Isoxazolochinolinon-Derivate der allgemeinen Formel I

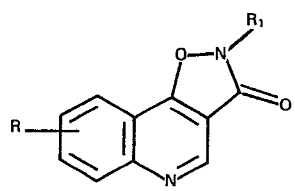

(I)

worin R ein Wasserstoff-, Fluor-, Chlor- oder Bromatom, einen linearen Alkylrest mit 1 bis 5 Kohlenstoffatomen oder einen verzweigten Alkylrest mit 3 bis 5 Kohlenstoffatomen, einen Alkoxyrest mit 1 bis 5 Kohlenstoffatomen, eine Nitrogruppe oder eine Trifluoromethylgruppe bedeutet und $R_1$ einen gegebenenfalls durch einen linearen Alkylrest mit 1 bis 5 Kohlenstoffatomen oder einen verzweigten Alkylrest mit 3 bis 5 Kohlenstoffatomen, durch eine Alkoxygruppe mit 1 bis 5 Kohlenstoffatomen oder durch ein Halogenatom substituierten Phenylrest bedeutet.

3. Derivate der allgemeinen Formel I gemäss Anspruch 1, dadurch gekennzeichnet, dass R ein Wasserstoff- oder Chloratom bedeutet.

3. 2-Phenyl-isoxazolo[4,5-c]chinolin-3(2H)-on.

4. 8-Fluor-2-phenyl-isoxazolo[4,5-c]chinolin-3-(2H)-on.

5. 8-Methoxy-2-phenyl-isoxazolo[4,5-c]chinolin-3(2H)-on.

6. Verfahren zur Herstellung der Isoxazolochinolinon-Derivate der allgemeinen Formel I gemäss Anspruch 1, dadurch gekennzeichnet, dass man ein Derivat der Formel II

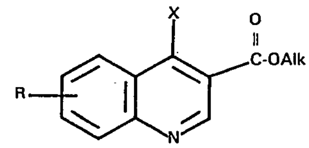

(II)

worin Alk für einen Methyl-, Ethyl- oder Propylrest steht, X ein Chlor- oder Bromatom darstellt und R die angegebene Bedeutung besitzt, mit einen Hydroxylamin-Derivat der Formel III

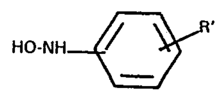

(III)

worin R' die für die etwaigen Substituenten des Phenylrestes in Anspruch 1 angegebene Bedeutung besitzt, umsetzt, um ein Derivat der allgemeinen Formel I zu erhalten, welches man isoliert.

7. Verfahren zur Herstellung von Isoxazolochinolinon-Derivaten der allgemeinen Formel I gemäss Anspruch 1, dadurch gekennzeichnet, dass man ein Derivat der Formel IV

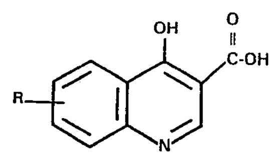

(IV)

worin R die angegebene Bedeutung besitzt, mit einem Hydroxylamin-Derivat der Formel III gemäss Anspruch 6 umsetzt, um ein Derivat der Formel V

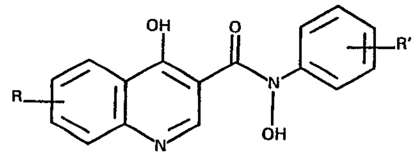

(V)

zu erhalten, worin R und R' die angegebene Bedeutung besitzen, welches man cyclisiert, um ein Derivat der allgemeinen Formel I zu erhalten.

8. Arzneimittel, dadurch gekennzeichnet, dass sie aus den neuen Isoxazolochinolinon-Derivaten der allgemeinen Formel I gemäss Anspruch 1 bestehen.

9. Arzneimittel, dadurch gekennzeichnet, dass sie aus den neuen Isoxazolochinolinon-Derivaten gemäss einem der Ansprüche 2 bis 5 bestehen.

10. Pharmazeutische Zusammensetzungen, dadurch gekennzeichnet, dass sie als Wirkstoff zumindest eines der Arzneimittel gemäss einem der Ansprüche 8 oder 9 enthalten.

11. Produkt der Formel V gemäss Anspruch 7.

**Claims**

1. Derivatives of isoxazoloquinolinone characterized in that they answer to the general formula I

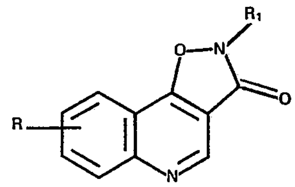

(I)

in which R represents a hydrogen, fluorine, chlorine or bromine atom, a linear alkyl radical containing from 1 to 5 carbon atoms, or branched alkyl radical containing from 3 to 5 carbon atoms, an alkoxy radical containing from 1 to 5 carbon atoms, a nitro or trifluoromethyl radical and $R_1$ represents a phenyl radical possibly substituted by a linear alkyl radical containing from 1 to 5 carbon atoms, or a branched alkyl radical containing from 3 to 5 carbon atoms, by an alkoxy radical containing from 1 to 5 carbon atoms, or by a halogen atom.

2. Derivatives as defined by the general formula I of claim 1, characterized in that R represents a hydrogen or chlorine atom.

3. 2-phenyl-isoxazolo(4,5-c)quinolin-3(2H)-one.

4. 8-fluoro-2-phenyl-isoxazolo(4,5-c)quinolin-3-(2H)-one.

5. 8-methoxy-2-phenyl-isoxazolo(4,5-c)quinolin-3(2H)-one.

6. Preparation process for derivatives of isoxazoloquinolinone as defined by the general formula I of claim 1, characterized in that a derivative with the formula II

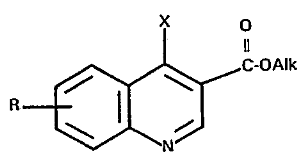

(II)

in which Alk represents a methyl, ethyl or propyl radical, X represents a chlorine or bromine atom, and R has the significance already indicated, is made to react with a derivative of hydroxylamine with the formula III

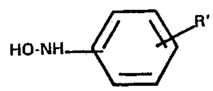

(III)

in which R' has the significance already indicated in claim 1 for the optional substitutions of the phenyl radical, in order to obtain a derivative with the general formula I, which is isolated.

7. Preparation process for derivatives of isoxazoloquinolinone as defined by the general formula I of claim 1, characterized in that a derivative with the formula IV

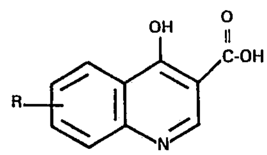

(IV)

in which R has the significance already indicated, is made to react with a derivative of hydroxylamine with the formula III, as defined in claim 6, in order to obtain a derivative with the formula V

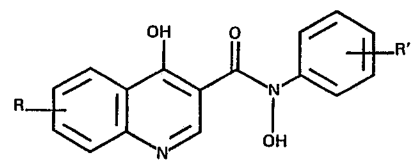

(V)

in which R and R' have the significance already indicated, which is cyclized in order to obtain a derivative with the general formula I.

8. Medicaments, characterized in that they are constituted by new derivatives of isoxazoloquinolinone, as defined by the general formula I of claim 1.

9. Medicaments, characterized in that they are constituted by new derivatives of isoxazoloquinolinone, as defined in one of the claims 2 to 5.

10. Pharmaceutical compositions, characterized in that they contain, as active principle, one at least of the medicaments as defined in one of the claims 8 or 9.

11. Products with the formula V, as defined in claim 7.